(19) Europäisches Patentamt
European Patent Office
Office européen des brevets

(11) EP 4 371 971 A1

(12) EUROPEAN PATENT APPLICATION

(43) Date of publication:
**22.05.2024 Bulletin 2024/21**

(21) Application number: **22306705.9**

(22) Date of filing: **18.11.2022**

(51) International Patent Classification (IPC):
***C07C 45/28** (2006.01)* ***C07C 47/58** (2006.01)*

(52) Cooperative Patent Classification (CPC):
(C-Sets available)
**C07C 45/28** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Deasyl SA**
**1228 Plan-les-Ouates (CH)**

(72) Inventors:
- **Rodriguez-Padron, Daily**
  **30175 Marghera (IT)**
- **Halloumi, Samy**
  **74380 Nangy (FR)**
- **Malpartida Garcia, Irene**
  **29649 Mijas costa (ES)**
- **Lair, Valentin**
  **74100 Annemasse (FR)**
- **Thiel, Julien**
  **74930 ARBUSIGNY (FR)**
- **Luque, Rafael**
  **14001 Córdoba (ES)**
- **Lacoste, François**
  **92200 NEUILLY SUR SEINE (FR)**

(74) Representative: **Jacobacci Coralis Harle**
**32, rue de l'Arcade**
**75008 Paris (FR)**

# (54) METHOD FOR MANUFACTURING SYNTHETIC VANILLIN

(57) The invention relates to method for manufacturing vanillin that comprises at least the following steps: (A) placing at least a specific starting material in a solvent with a liquid oxidant, referred to as "the starting mixture", the molar ratio of (liquid oxidant):( starting material) being greater than or equal to 2; (B) milling said starting mixture at a temperature lower than or equal to 80°C, in a three-dimensional liquid-phase microbead mill for a holding time lower than or equal to 180 min, (C) recovering, at the outlet of the mill, a final product comprising at least vanillin and optionally other side-products.

EP 4 371 971 A1

(52) Cooperative Patent Classification (CPC): (Cont.)

C-Sets
**C07C 45/28, C07C 47/58;**
**C07C 45/28, C07C 49/255**

## Description

### TECHNICAL FIELD OF THE INVENTION

The invention relates to a method for manufacturing synthetic vanillin.

In particular, the present invention refers to a method for synthetizing vanillin, performed by mechanochemistry from a starting mixture comprising at least a specific precursor, preferably selected from a biomass-derived molecule, and a liquid oxidant.

### BACKGROUND INFORMATION AND PRIOR ART

Vanillin is the most common flavour chemical used in a broad range of flavors and fragrances and is hence commonly used in flavourings, food and perfumes. Vanillin may also be used in the pharmaceutical field. For instance, it may be used as a chemical intermediate in the manufacture of several drugs and other products. It also has medicinal uses as an anticlastogenic and an antimicrobial agent.

Vanillin (CAS 121-33-5) is an organic compound, specifically a phenolic aldehyde. Its functional groups include aldehyde, hydroxyl, and ether. It has the following formula (I):

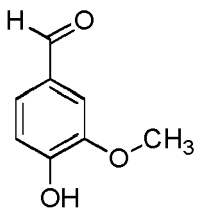

(I)

Vanillin is both naturally occurring and synthetically produced.

It can be isolated from natural source derived extracts, including vanilla beans and pods, pine woods (e.g., *Pinus tabuliformis*) and roasted coffee. However, the increasing global demand of vanillin cannot be met by the supply of vanilla orchid pods as the sole source. Only ca. 1 % of the global production of vanillin is derived from vanilla pods.

Because of the scarcity and expense of natural vanilla extract, synthetic preparation of its predominant component has long been of interest. The first commercial synthesis of vanillin began with the more readily available natural compound eugenol (4-allyl-2-methoxyphenol). Also, artificial vanillin is made either from guaiacol or lignin. For instance, vanillin can be obtained from lignin fractions through depolymerization and subsequent conversion and/or isolation.

Indeed, various processes for the manufacture of vanillin have been proposed in the prior art.

A way to produce synthetic vanillin is by chemical synthesis. As mentioned-above, vanillin may be synthesized from eugenol. In general, for this reaction, the use of a catalyst is essential. Also, vanillin may be synthesized from guaiacol. At present, the most significant of these is the two-step process, in which guaiacol reacts with glyoxylic acid by electrophilic aromatic substitution. The resulting vanillylmandelic acid is then converted by 4-hydroxy-3-methoxyphenylglyoxylic acid to vanillin by oxidative decarboxylation.

Hence, up to now, the downside of the production of synthetized vanillin by chemical way is the use of petro-based intermediates, such as glyoxylic acid and guaiacol and the use of no eco-friendly materials, such as some non-recyclable catalysts. In addition, it has been determined that the production of vanillin from petroleum-intermediate (Riedel process) is not only not environmentally friendly, but also are of poor quality.

Some alternatives have been reported in the literature for the synthesis from biomass-derived platform molecules. However, in many cases those alternatives involve high temperatures (up to 90 °C) or long reaction times (up to 24 h), and in all cases the use of a catalytic system is required. (Rodriguez-Padron, D., Munoz-Batista, M. J., Li, H., Shih, K., Balu, A. M., Pineda, A., Luque, R. ACS Sustain. Chem. Eng., 2019, 7(20), 17030-17038).

Hence, an object of the current invention is thus to propose a new method to synthetize vanillin which avoids, at least in part, the aforementioned drawbacks.

In particular, an object of the current invention is to provide a novel chemical method for synthetizing vanillin that enables to produce vanillin having a good selectivity, while being at the same time not or few expensive, not time consuming, with no or fewer difficulties in purification and this with an appropriate conversion rate.

Another object of the current invention is to provide a green chemical method for synthetizing vanillin that efficiently utilize resources and raw feedstocks, reduce waste streams, and avoid the use of toxic and hazardous materials.

## SUMMARY OF THE INVENTION

The Applicant sought to develop a new chemical method for synthetizing vanillin that enables to obtain vanillin having good quality, while being industrially exploitable, easy to implement and with an appropriate good conversion rate.

For that purpose, the Applicant discovered that the use of a three-dimensional microbead mill allows to perform vanillin synthesis, preferably, from biomass-derived compounds and offers significant advantages with respect to batch reactor designs, such as high control over reaction conditions, to work under a continuous operational mode, well-defined properties, excellent safety, etc.

The invention relates therefore to a method for manufacturing vanillin that comprises at least the following steps:

(A) placing at least a starting material having the following formula (I):

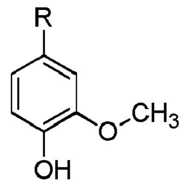

(I)

wherein **R** is selected from the group consisting of:

- a unsaturated straight or branched hydrocarbon group containing 1 to 3 carbon atoms, such as - $CH=CH-CH_3$;
- $R^1COOH$ wherein $R^1$ is a unsaturated, straight or branched hydrocarbon group containing 1 to 3 carbon atoms, such as -CH=CH-COOH,
- $R^2OH$ wherein $R^2$ is a unsaturated, straight or branched hydrocarbon group containing 1 to 3 carbon atoms, such as $-CH_2-OH$,

in a solvent with a liquid oxidant, refered to as "the starting mixture", the molar ratio of (liquid oxidant):(starting material) being greater than or equal to 2, preferably being within the range from 5 to 25, and in particular being within the range from 5 to 15;

(B) milling said starting mixture at a temperature lower than or equal to 80°C, preferably lower than or equal to 55°C, in a three-dimensional liquid-phase microbead mill for a holding time lower than or equal to 180 minutes, preferably lower than or equal to 30 minutes, and in particular lower than or equal to 10 minutes,

(C) recovering, at the outlet of the mill, a final product comprising at least vanillin and optionally other side-products.

The invention also refers to the use of a three-dimensional microbead mill such as described above to synthetize vanillin.

## Brief description of the drawings

For a more complete understanding of the description provided herein and the advantages thereof, reference is now made to the brief description below, taken in connection with the accompanying drawings and detailed description.

Fig.1 represents a view in cross section along the longitudinal axis XX of a three-dimensional wet-phase microbead mill, according to one implementation variant that is suitable for performing the process according to the invention;

Fig.2 represents views in cross section along the axis XX and the axis AA, of variants of three-dimensional wet-phase microbead mills according to figure 1 in which: (a) the agitator is a disk agitator, (b) the agitator comprises fingers and (c) the milling chamber is annular;

FIG. 3 shows the CG-MS spectra of the obtained products from the oxidation of isoeugenol into a three-dimensional liquid-phase microbead mill according to an embodiment of the invention.

## DETAILED DESCRIPTION OF THE INVENTION

### A) Definitions

The terms "comprise" (and any grammatical variation thereof, such as "comprises" and "comprising"), "have" (and any grammatical variation thereof, such as "has" and "having"), "contain" (and any grammatical variation thereof,

such as "contains" and "containing"), and "include" (and any grammatical variation thereof, such as "includes" and "including") are open-ended linking verbs. They are used to specify the presence of stated features, integers, steps or components or groups thereof, but do not preclude the presence or addition of one or more other features, integers, steps or components or groups thereof. As a result, a method, or a step in a method, that "comprises," "has," "contains," or "includes" one or more steps or elements possesses those one or more steps or elements but is not limited to possessing only those one or more steps or elements.

Unless otherwise indicated, all numbers or expressions referring to quantities of ingredients, ranges, reaction conditions, etc. used herein are to be understood as modified in all instances by the term "about."

Also, unless otherwise indicated, the indication of an interval of values « from X to Y » or "between X to Y", according to the present invention, means as including the values of X and Y.

**B) Method for synthetizing vanillin**

The Applicant has developed a new green chemical method so as to produce synthetic vanillin. Especially, the Applicant has developed a method which, surprisingly and unexpectedly, allows the synthesis of vanillin at ambient temperature (i.e.:25°C), in a single milling step, and this in very short times (the residence time of the reagents in the three-dimensional liquid-phase microbead mill is lower than or equal to 180 minutes versus 24 hours for the process of the prior art using isoeugenol as starting material (such as the publication of Rodriguez-Padron et al., ACS Sustain.Chem.Eng., 2019, 7(20), 17030-17038).

As it will be demonstrated in the experimental tests below, the method of the invention using a particular mill, namely a three-dimensional liquid-phase microbead mill, combined with particular reaction conditions (choice and molar concentration of the starting material and liquid oxidant, flow rate, etc.) makes it possible to manufacture vanillin with a conversion rate of greater than or equal to 50% and in particular greater than or equal to 70% with a residence time of at least 5 minutes and less than or equal to 180 minutes.

The method according to the invention also has the advantages of having a very low-cost price (the raw materials used are in fact available, non-polluting and inexpensive) and of having an excellent reproducibility. The method according to the invention also has the advantage of being able to be carried out continuously. These characteristics are important for industrial-scale application.

In addition, despite the extensive research carried out on the synthesis of vanillin during the past few decades (via chemical, biochemical, enzymatic methods or by the biotechnology-based approach), none has suggested the claimed method and in particular a milling step in a three-dimensional microbead mill from the starting reagents.

Hence, the present invention relates to a method for manufacturing vanillin that comprises at least the following steps:

(A) placing at least a starting material having the following formula (I):

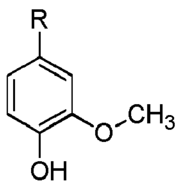

(I)

wherein R is selected from the group consisting of:

- a unsaturated straight or branched hydrocarbon group containing 1 to 3 carbon atoms, such as - $CH=CH-CH_3$;
- $R^1COOH$ wherein $R^1$ is a unsaturated, straight or branched hydrocarbon group containing 1 to 3 carbon atoms, such as -CH=CH-COOH,
- $R^2OH$ wherein $R^2$ is a unsaturated, straight or branched hydrocarbon group containing 1 to 3 carbon atoms, such as $-CH_2-OH$,

in a solvent with a liquid oxidant, refered to as "the starting mixture", the molar ratio of (liquid oxidant):(starting material) being greater than or equal to 3, preferably being within the range from 5 to 25, and in particular being within the range from 5 to 15;

(B) milling said starting mixture at a temperature lower than or equal to 80°C, preferably lower than or equal to 55°C, in a three-dimensional liquid-phase microbead mill for a holding time of lower than or equal to 180 min, preferably lower than or equal to 30 min, and in particular lower than or equal to 10 min,

(C) recovering, at the outlet of the mill, a final product comprising at least vanillin and optionally other side-products.

Especially, the method according to the invention enables to perform the following synthesis:

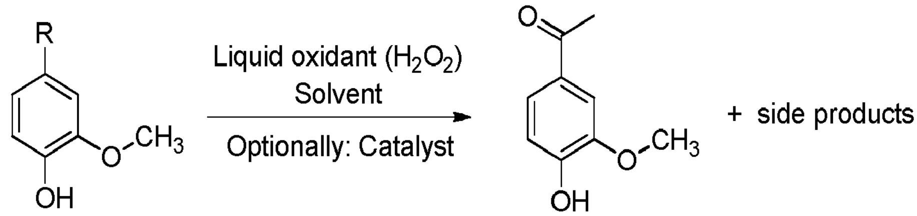


Preferably, the starting material is a natural compound or is derived from a natural compound that is preferably renewable.

In general, the starting material is selected from the group consisting of isoeugenol (R= $-CH=CH-CH_3$), vanillyl alcohol (R= $CH_2-OH$), ferulic acid (R= -CH=CH-COOH) and combination thereof and is typically isoeugenol.

In particular, a suitable liquid oxidant according to the invention may be selected from the group consisting of: hydrogen peroxide ($H_2O_2$), urea hydrogen peroxide ($CO(NH_2)_2*H_2O_2$, CAS 124-43-6), tert-butyl hydroperoxide ($(CH_3)_3COOH$) and a combination thereof. Preferably, the liquid oxidant is hydrogen peroxide ($H_2O_2$).

Hence, the suitable liquid oxidant according to the invention is a green oxidant to respect the environmental concerns.

In particular, the solvent may be selected from acetonitrile, methanol, ethanol and is preferably acetonitrile.

According a first embodiment of the invention, the starting mixture at step (A) comprises at least a catalyst.

The catalyst may be selected from metals-based catalysts, such as transition-based catalyst as Fe, Ni, Co, Pt, Pd and may be preferably Fe-based catalysts, such as a Feoxides.

According to another embodiment of the invention, the starting mixture at step (A) is free from any catalyst. Preferably, the starting mixture at step (A) is free from any catalyst.

The Applicant noticed that most of the prior art documents relating to the production of vanillin requires the presence of a catalytic system. However, according to the invention, the presence of a catalyst is not essential. Surprisingly, the Applicant discovered that vanillin reaction according to the method of the invention may be carried out in absence of such a catalyst and gives rise to remarkable results, most likely attributed to the effective microbeads-assisted mechanical approach.

Hence, the method according to the invention employs not only a renewable feedstock derived from biomass as starting material (such as isoeugenol), but also reduces the use of non-recyclable material, such as catalyst. This enables a consequent reduction of the cost and reactants involved in the preparation of a catalyst.

**B1)** Suitable three-dimensional microbead mill

To better understand the process that is the subject of the invention, a three-dimensional microbead mill capable of allowing the production of vanillin, and thus forming part of the invention, will first be described below with reference to figures 1 and 2.

As illustrated in Fig.1, a three-dimensional microbead mill 1 comprises at least:

- a stationary milling chamber 2 of cylindrical general shape extending along a longitudinal axis XX, said chamber 2 being at least partly filled with said microbeads (not shown) and comprises: at a first end 3 at least one inlet 5 serving to introduce said starting mixture (i.e.: starting material such as isoeugenol and the liquid oxidant, such as $H_2O_2$), and at a second end 4, an outlet 6 comprising a separating means 7 that is capable of evacuating only the vanillin and the optionally other side-products thus formed in said chamber 2; and
- an agitator 8, arranged in the stationary milling chamber 2, which is in the form of a rod elongated along the longitudinal axis XX, said agitator 8 being capable of placing the microbead/starting mixture assembly in motion.

In particular, the inlet 5 is generally connected to a peristaltic pump (not shown). This pump delivers the starting material contained, for example, in a container, such as a tank, into the milling chamber 2 via the inlet 5. The pump also makes it possible, during the functioning of the three-dimensional mill, to convey the starting material and the liquid oxidant at a certain flow rate that is adjustable, referred to hereinbelow as the "passage flow rate". This passage flow rate also forms a stream in the milling chamber 2 for entraining the starting mixture from the inlet 5 to the outlet 6.

The outlet 6 of the milling chamber 2 in particular comprises the system 7 for separating the microbeads from the final product predominantly comprising the vanillin and the optionally other side-products thus formed and/or the

remaining unreacted starting materials. This separating means 7 may be a screen whose orifices have a size smaller than that of the microbeads or a separating slit whose width is also adapted to retain the microbeads in the chamber 2.

The inner wall 9 of the milling chamber 2 comprises, according to a first embodiment, a smooth inner surface. However, according to an implementation variant that will be described below, fingers 11 may be arranged on this inner surface 9.

As mentioned above, the agitator 8 is placed inside the milling chamber 2 and, in addition to the passage flow rate, said agitator also places the starting mixture in motion.

In particular, the agitator 8 is capable of rotating about the axis X via a rotary shaft (14, figure 2) to give the starting mixture, in the milling chamber 2, a turbulent motion and thus to effect intense blending between this starting mixture and the microbeads present in the chamber 2 along the inner wall 9 of this chamber 2.

In order to improve this blending, the agitator 8, and similarly the inner wall 9 of the chamber 2, may have various possible configurations represented, for example, in figure 2.

According to a first configuration illustrated in figure 2a, the agitator 8 comprises along its elongated rod disks 10 arranged perpendicular thereto. They may vary in number from 2 to 8, preferably from 2 to 5. These disks 10 make it possible, firstly, to improve the milling of the starting mixture by better blending the microbeads, and, secondly, to accelerate the reaction time.

According to a second configuration illustrated in figure 2b, the agitator 8 may also comprise along its rod one or more disks 10 arranged perpendicularly, and which are also capable of cooperating with fingers 11 arranged perpendicularly, relative to the inner wall 9 of the chamber 2. A finger is especially in the form of a ring which extends perpendicularly from the wall 9. For this configuration, the disks 10 and the fingers 11 are arranged in a staggered manner, i.e., the disks 10 and the fingers 11 are arranged alternately in the chamber 2. In addition, the thickness of the rod 8 is increased relative to the preceding configuration (figure 2a) so that the periphery of the disks 10 is close to the inner wall 9 and so that the periphery of the fingers 11 is close to the periphery of the rod of the agitator 8. Thus, in this configuration, the volume of the chamber is reduced relative to the preceding configuration, consequently allowing better blending between the starting mixture, the microbeads and the inner wall 9 of the chamber 2.

The volume of the chamber 2 may also be reduced as is illustrated in figure 2c. In this configuration, the agitator 8 has an outside diameter slightly smaller than the inside diameter of the chamber 2, thus forming an annular chamber 12 of low volume arranged between the outer wall of the agitator 8 and the inner wall 9 of the chamber 2. The microbeads (not shown) are placed in this annular chamber 12. During the functioning of this configuration, the starting mixture is introduced via the inlet 5 at a certain flow rate, and then passes through the annular chamber 12 up to the outlet 6 while at the same time being blended by the microbeads.

In general, the mill that is suitable for performing the process according to the invention comprises a milling chamber with a diameter of from 75 mm to 300 mm for a length of from 80 mm to 900 mm and an agitator ranging from 60 mm to 260 mm in size. Thus, the volume of the milling chamber ranges from 0.35 L to 600 L, preferably from 0.35 L to 62 L.

The geometry of the milling chamber and of the agitator may be adjusted by a person skilled in the art as a function of the desired amount of vanillin, and also of the desired reaction time. For example, it is also possible for the milling chamber 2 to comprise an accelerator so as to improve the milling of the starting mixture.

In addition, the microbeads housed in the milling chamber 2, and which are suitable for use in the process according to the invention, are substantially spherical and have a mean diameter ranging from 0.05 mm to 4 mm, preferably from 0.2 to 3 mm, in particular from 0.3 to 2 mm, and typically about 0.5 to 1 mm. Preferably, the diameter of the microbeads is lower than or equal to 1 mm.

They are preferentially chosen from microbeads of high hardness and relatively good resistance to abrasion.

In particular, the microbeads have a Vickers hardness measured according to standard EN ISO 6507-1 of greater than or equal to 900 HV1, preferably ranging from 900 HV1 to 1600 HV1, typically ranging from 1000 to 1400 HV1.

Advantageously, they have a high real mass per unit volume. In general, the microbeads according to the invention have a real mass per unit volume of greater than or equal to 2 g/cm$^3$, in particular ranging from 2 to 15 g/cm$^3$, preferably from 3 to 12 g/cm$^3$, and typically from 4 to 10 g/cm$^3$.

Thus, the microbeads according to the invention may be ceramic microbeads (zirconium oxide $ZrO_2$, zirconium silicate $ZrSiO_4$); steel microbeads, tungsten carbide microbeads, glass microbeads or a combination thereof.

Preferably, the microbeads are ceramic since they do not generate any pollution as a result of their wear.

In particular, the microbeads are made of zirconium oxide.

Optionally, the zirconium oxide microbeads may be stabilized with another oxide, such as cerium oxide, yttrium oxide and/or silicon.

By way of examples, the following compositions, summarized in Table 1 below, are suitable for forming the microbeads according to the invention:

*Table 1*

| Composition of the microbeads | HV1 hardness | Real mass per unit volume ($g/cm^3$) | Manufacturer |
|---|---|---|---|
| Zirconium oxide microbeads stabilized with cerium oxide<br>80% $ZrO_2$<br>20% CeO | 1180 | ≥ 6.10 | Saint-Gobain (Zirmil®Y Ceramic Beads) or EIP (Procerox® ZO Cer) |
| Zirconium oxide microbeads stabilized with yttrium<br>95% $ZrO_2$<br><5% $Al_2O_3$<br>Remainder: $Y_2O_3$ | 1250 | ≥ 5.95 | EIP (Procerox® ZO (Y)) |
| Zirconium oxide microbeads stabilized with yttrium and silicon:<br>78% $ZrO_2$,<br>12% $SiO_2$,<br>5% $Al_2O_3$ and<br>4% $Y_2O_3$ | > 700 | >4.80 | Saint-Gobain (ER120 Ceramic Beads) |
| Zirconium silicate $ZrSiO_4$ microbeads | > 800 | > 6.5 | Saint-Gobain (Rimax Ceramic Beads) |
| Glass microbeads | 500 | > 3.76 | - |
| Steel microbeads | 700 | > 7.7 | - |

In particular, the microbeads represent, by volume relative to the total volume of the stationary chamber 2, from 20% to 90%, preferably from 40% to 85%, and in particular from 55% to 70%.

As used herein, "a range from 20% to 90%" covers the following values or any interval between the following values: 20; 25; 30; 35; 40; 45; 50; 55; 60; 65; 70; 75; 80; 85; 90.

By way of example, the three-dimensional wet-phase microbead mill that is suitable for performing the process according to the invention may correspond to mills sold by the companies WAB, Dyno-Mill range: Multi Lab, ECM and KD, Netzsch, for example Labstar LS1, or Alpine Hosokawa, for example Agitated Media Mill AHM.

**B2)** Description of the method according to the invention

The method for synthetizing vanillin according to the invention will now be described more explicitly below.

As indicated previously, the manufacture of vanillin according to the invention comprises a first step (A) of placing at least a starting material having the following formula (I):

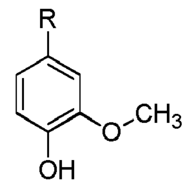

(I)

wherein R is selected from the group consisting in:

- a unsaturated straight or branched hydrocarbon group containing 1 to 3 carbon atoms, such as - $CH=CH-CH_3$;
- R1COOH wherein R1 is an unsaturated, straight or branched hydrocarbon group containing 1 to 3 carbon atoms, such as -CH=CH-COOH,
- $R^2OH$ wherein $R^2$ is a unsaturated, straight or branched hydrocarbon group containing 1 to 3 carbon atoms, such as $-CH_2-OH$,

in a solvent with a liquid oxidant.

The mixture obtained is referred hereinbelow as "starting mixture".

According to an embodiment of the invention, the manufacture of vanillin may include a preliminary step (0) of preparing the starting mixture. It is indeed generally easier from a practical point of view to prepare the starting mixture including the different starting reagents.

For instance, the starting mixture may be conventionally prepared by mixing the starting reagents (i.e.: stating material of formula (I), solvent and liquid oxidant) in a suitable device, such as a container or a tank, equipped with a stirring system (such as a magnetic stirrer, stirring paddles, etc.). The device and the stirring system may be adapted by a person skilled in the art as a function of the amount of vanillin to be manufactured. Optionally, when it is present, the catalyst can be added later in the three-dimensional mill 100.

According to the invention, the molar ratio of (liquid oxidant):( starting material) is greater than or equal to 2, preferably ranges from 5 to 25, and in particular ranges from 5 to 15.

For the purposes of the invention, "a molar ratio greater than or equal to 2" covers the following values or any interval between the following values: 2; 3; 4; 5; 6; 7; 8; 9; 10; 11; 12; 13; 14; 15; etc.

Hence, this excess of liquid oxidant allows not only the synthesis of the vanillin, but also promotes the placing in motion of the microbeads of the mill for better milling of the starting mixture and thus better synthesis of vanillin.

According to the invention, the molar concentration of starting material in the solvent is of at least 0.05 mol/L, preferably is within the range from 0.05 to 10 mol/L, and in particular within the range from 0.2 to 0.3 mol/L. For instance, there is 0.3 mol of starting material for 1 L of solvent.

As used herein, "a range of at least 0.005 mol/L covers the following values or any interval between the following values: 0.05; 0.06; 0.07; 0.08; 0.09; 0.1; 0.5; 1.0; 1.5; 2.0; 2.5; 3; 3.5; 4.0; 4.5; 5.0; 5.5; 6.0; 6.5; 7.0; 7.5; 8.0; 8.5; 9.0; 9.5; 10.0; 10.5; etc.

Once the starting mixture is prepared, it is conveyed to the three-dimensional microbead mill 1 generally by means of the peristaltic pump at a flow rate that is adjustable via the inlet 5. The peristaltic pump allows mixing of the starting mixture to be continued before entry into the chamber 2. In addition, as indicated previously, this pump makes it possible to introduce the starting mixture into the chamber 2 at a controlled passage flow rate.

Generally, for a microbead mill comprising a stationary milling chamber having a size ranging of 0,5 L, the starting mixture is introduced at a passage flow rate from 0,01 to 200 Uh, preferably from 0,03 to 100 L/h and typically from 0,05 to 10 L/h. For bigger microbead mill comprising for instance a stationary milling chamber having a size ranging of 10 L, the starting mixture may be introduced at a passage flow rate from 0,01 to 1000L/h, preferably from 0,5 to 500 L/h and typically from 1 to 100 Uh.

Once the starting mixture is introduced into the chamber 2, the milling step (B) begins.

Under the effect of the stream created by the passage flow rate, the starting mixture travels through the stationary chamber 2 from the inlet 5 to the outlet 6, while at the same time being placed in motion by the agitator 8 which allows intense blending of this mixture with the microbeads and, where appropriate, with the disks 10, the fingers 11, etc., along the inner wall 9 of the chamber 2.

The rotation speed of the agitator may range, for example, from 4 to 20 Pi rad/s, preferably from 4 to 8 Pi rad/s.

The holding time of the starting mixture into the three-dimension microbead mill is lower than or equal to 180 minutes, preferably lower than or equal to 30 minutes, and ranges especially from 5 to 10 minutes.

For the purposes of the invention, "an holding time lower than or equal to 180 minutes" covers the following values or any interval between the following values: 180; 175; 170; 165; 160; 155; 150; 145; 140; 135; 130; 125; 120; 115; 110; 105; 100; 95; 90; 85; 80; 75; 70; 65; 60; 55; 50; 45; 40; 35; 30; 25; 20; 19; 18; 17; 16; 15; 14; 13; 12; 11; 10; 9; 8; 7; 6; 5; 4; 3; 2; 1; 0,5 minute(s).

This holding time is in fact inherent to the apparent volume of the microbeads and to the passage flow rate. For example, if the total apparent volume of the beads is 270 $cm^3$ (microbeads with an apparent mass per unit volume of 3.7 $g/cm^3$) and if the rate of introduction of the mixture is 30 L/h, i.e., 8.3 $cm^3/s$, then the residence time of the mixture in the chamber 2 is estimated at about 32 seconds. Consequently, the residence time may be advantageously adjusted, for example, by controlling the apparent mass per unit volume of the microbeads, and also the passage flow rate.

The term "apparent volume" means the volume of the microbeads including the interstitial air between the beads. The apparent mass per unit volume is the ratio between the mass of the microbeads and the apparent volume.

The milling step may be performed in continuous mode or in batch mode.

In particular, the continuous mode corresponds in general to a recirculation mode wherein the mixture recovered at the outlet 6 of the three-dimensional liquid-phase microbead mill is reinjected again, by means of the pump, into the chamber 2 via the inlet 5 to allow another pass. Hence, the continuous mode comprises a single pass or several successive passes respecting the total residence time.

For this recirculation/continuous mode, the number of passes of the "starting" mixture, depending on the total residence time, may be from 1 to 50, preferentially from 1 to 30. In particular, the number of passes of the starting mixture may be 10.

Specifically, the Applicant has noted that a single pass in the microbead mill, despite a very short residence time, made it possible to obtain at the outlet 6 a final mixture comprising predominantly vanillin of very satisfactory quality.

Thus, the milling step will preferably be performed in continuous mode.

The batch mode corresponds in general to the embodiment wherein the outlet 6 and the inlet 5 of the three-dimensional liquid-phase microbead mill several are not linked together by a pump, but several passes are also performed.

This milling step proceeds at a temperature of lower than or equal to 80°C, preferably lower than or equal to 55°C, i.e., usually at temperature ranging from 15°C to 80°C, preferably ranging from 15°C to 55°C, and typically ranging from 15°C to 50°C and in general at the ambient temperature, i.e.: about 20°C to 55°C, such as 20°C to 40°C.

As used herein, "a temperature ranging from 15°C to 80°C" covers the following values or any interval between the following values: 15; 16; 17; 18; 19; 20; 21; 22; 23; 24; 25; 26; 27; 28; 29; 30; 31; 32; 33; 34; 35; 36; 37; 38; 39; 40; 45; 50; 55; 60; 65; 70; 75; 80.

Specifically, the process according to the invention does not require any particular heating in order to manufacture the vanillin (i.e.: is performed at the temperature of the room wherein the three-dimensional liquid-phase microbead mill is implemented), in contrast with some of the teachings disclosed in the prior art. Hence, in general, the milling step proceeds at ambient temperature (i.e.: 20°C to 40°C).

Once the milling step has been performed, a final product comprising at least vanillin and optionally other side products is recovered at the outlet 7 of the mill 100 (step (C)).

This final product may comprise traces of the starting reagents which have not reacted, such as, for example, the catalyst or alternatively a by-product, namely vanillin methyl ketone, eugenol and/or dihydrodiisoeugenol if the starting material is isoeugenol and if the reaction temperature or the concentrations of the starting reagents were not in the ideal reaction ranges.

The method according to the invention enables to obtain vanillin with an appropriate yield, especially higher than or equal to 35%, preferably higher than or equal to 40% and in particular higher than or equal to 45% for a residence time of 30 minutes.

Especially, the method according to the invention enables to obtain vanillin with a suitable conversion rate. For instance, the conversion rate (%) of vanillin manufactured with the method according to the invention is higher than or equal to 50%, preferably higher than or equal to 70% and especially higher than or equal to 80% for a residence time of 60 minutes.

For the purposes of the invention, "a conversion rate (%) of vanillin higher than or equal to 50%" covers the following values or any interval between the following values: 50; 51; 52; 53; 54; 55; 56; 57; 58; 59; 60; 61; 62; 63; 64; 65; 66; 67; 68; 69; 70; 71; 72; 73; 74; 75; etc.

In a known manner, the vanillin may be separated from the reaction medium (from the starting reagents, i.e.: the remaining optional catalyst, the remaining starting material, the remaining liquid oxidant) or even from the other by-product(s) formed) by methods well known to those skilled in the art. These methods may be, for example, by extraction or by chromatographic methods.

Vanillin can also be purified, if necessary, by techniques that are well known to the skilled person in the art, such as chromatographic methods.

## EXAMPLES

The description of the tests below is given by way of purely illustrative and nonlimiting example.

For the following experiments, the starting reagents are illustrated in the table 2 below:

*Table 2*

| | Product/Trademark | Supplier |
|---|---|---|
| **Starting material** | Isoeugenol | Sigma-Aldrich |
| **Liquid oxidant** | $H_2O_2$ | Sigma-Aldrich |
| **Catalyst** | $Fe_2O_3$ | According to ES2717979 |

The tests were carried out in a three-dimensional micro-bead mill Dynomill ECM AP-05 of Willy A. Bachofen AG (WAB), which contains 1.235 kg of micro-beads and was adapted to include a heating device, such as described in the patent application WO2019/228983.

Namely, the mill comprises a heating device positioned at the entrance to the stationary chamber, and the first mixing element acts as a susceptor (patent FR18 54592).

In particular, the heating device has the following characteristics:

*Table 3*

| Elements | Characteristics |
|---|---|
| Generator | 10kW generator with a frequency ranging from 17 to 200 kHz/generator in series IDPartner reference IX3600 model PO8010. |
| Inductor | Multi-strand Litz wires, resinated to be removable. Cable from IDPartner 300 strands Litz Cu 9.425 $mm^2$ 6x50x0.2 mm. |
| Susceptor | Mixing element as described in US 5,597,126 (fig. 4) in stainless steel Phytem® 260 equivalent ferric stainless steel Kara from ArcelorMittal grade K44. |
| Magnetic shield: | Cylindrical torus in Fluxtrol® |
| Power supply means | Rod 11 has been modified to incorporate the 3 $mm^2$ copper coaxial power supply. This coaxial cable changes the centre of gravity of the rod; it is thus balanced by compensating it with tungsten screws. |
| Thermocouples | Type K at the inlet and the outlet of the milling chamber. |
| Contactor | Cooper rotary contactor. |

The microbeads are made of zirconium oxide and have a diameter of 0.5 mm. The characteristics of the microbeads used for the tests are summarized in Table 4 below:

Table 4

| **Microbeads** | 0.45/0.55 mm |
|---|---|
| **Composition (% per mass)** | 93% ZrO2<br>5% Y2O3<br>2% others |
| **Specific density** | 6.0 g/cc |
| **Apparent density** | 3.7Kg/L |
| **Vickers hardness** | 1250 HV1 |

The 0.5 mm microbeads are marketed under the brand name Zirmil® Y Ceramic Beads by the company Saint-Gobain.

The grinding chamber of the mill has a capacity of 514 mL and is filled, by volume, with respect to its total volume and according to the tests, with 167 or 334 mL of the microbeads described above.

In operation, the microbeads are stirred by the stirrer at a rotational speed which may vary according to the examples. The stirrer also comprises mixing accelerators made of steel alloy.

**Example 1** : **Influence of the milling step according to the invention**

So as to study the influence of the milling step according to the invention, a first example has been performed without using the three-dimensional microbead mill according to the invention.

*Preparation of the starting mixture*

A first mixture of isoeugenol (0.3M) and acetonitrile (1L) and a second mixture of $H_2O_2$ (0.2 mol) were prepared. Each mixture is stirred magnetically and vigorously at 25°C in a 1 liter round-bottomed three-necked flask. The molar ratio ($H_2O_2$:isoeugenol) is of 1:1 The first mixture is connected to a first pump and the second mixture is connected to a second pump, these first and second pump are linked by a Y connector at the inlet of the microbead mill. *Introduction to the three-dimensional mill:*

The first and second mixtures are introduced into the microbead mill thanks to the Y connector and form the starting mixture (the temperature of this starting mixture is of 25°C). No catalyst is used for performing this example 1. After a residence time of 5 min in the mill, a final product is obtained and is recovered from the mill. This final product is analyzed

as described below.

*Sample Analysis*

The analyze of the final product resulting from the reaction was carried out by gas chromatography in an Agilent 6890N gas chromatograph (Agilent, Santa Clara, CA, USA). The chain consists of a manual injection system equipped with a septum (SPI), a Supelco 2-8047-U capillary column (15m x0.25mm i.d. and 0.25°m film thicknesses, Alltech Part No.31163-01), an oven, a flame ionization detector (FID, 70 eV, 30000, and 250 °C) and an acquisition system.

*Result*

This experiment without the use of a three-dimensional microbead mill according to the invention enables to obtain a low vanillin conversion rate that is lower than 10%. As shown on Fig.3 and on the schematic representation below, under these conditions, isoeugenol was converted to vanillin as main product, and reveals the presence of vanillin methyl ketone, eugenol and dihydrodiisoeugenol as side-products.

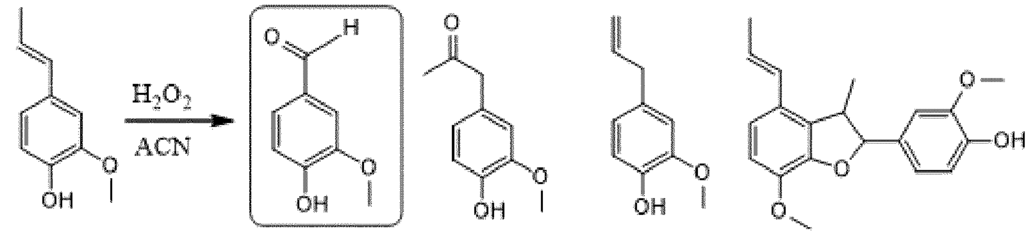


1 152,05 g/mol | 2 180,08 g/mol | 3 164,08 g/mol | 4 326,39 g/mol

**Example 2: influence of the temperature**

Isoeugenol oxidation has been investigated in the prior art literature at different reaction temperatures, ranging from room temperature up to 90 °C, displaying the best results either at high temperatures or by the combination of room temperature and long reaction times (up to 24 h) (Rodriguez-Padron, D., Munoz-Batista, M. J., Li, H., Shih, K., Balu, A. M., Pineda, A., Luque, R. ACS Sustain. Chem. Eng., 2019, 7(20), 17030-17038).

In this experiment, the conditions were similar to the ones described at Example 1, except the reaction temperatures.

Indeed, the reactions have been performed under continuous-flow regimes and the influence of the temperature in the reaction performance was investigated by carrying out experiments at 25 °C and 40 °C. Through this protocol, mechanochemical reactions occurred under controlled temperature. No catalyst is used for performing this example 2. After a residence time of 5, 30 and 60 minutes in the mill, a final product is obtained and is recovered from the mill.

*Results*

This vanillin conversion rate (%), the selectivity of the vanillin (%) and the vanillin yield (%) of the final products have been determined.

The conversion and selectivity were calculated from the chromatograms by

$$\text{Conversion (\%)} = [(C_{initial}-C_{final)/}C_{initial}] * 100 \qquad \text{Equation 1}$$

$$\text{Selectivity (\%)} = [C_{product} /(C_{initial}-C_{final})] * 100 \qquad \text{Equation 2}$$

Where $C_{Initial}$ and $C_{Final}$ are the concentrations of the reagents before and after the reaction, respectively and the $C_{Product}$ is the concentration of the product, as determined by Gas Chromatography (GC).

At 25°C

*Table 5*

| Residence time (min) | Conversion rate (%) | Selectivity to vanillin (%) | Vanillin yield (%) |
|---|---|---|---|
| **5** | 72.6 | 52.9 | 38.4 |
| **30** | 71.9 | 52.7 | 37.9 |
| **60** | 68.4 | 53.4 | 36.5 |

At 40°C

*Table 6*

| Residence time (min) | Conversion rate (%) | Selectivity to vanillin (%) | Vanillin yield (%) |
|---|---|---|---|
| **5** | 83 | 52 | 43 |
| **30** | 84 | 58 | 49 |
| **60** | 92 | 50 | 46 |

It was observed that by performing the reaction, in absence of catalysts and at 25 °C, conversion values around 70% were achieved, with selectivity and yield values of vanillin of 53% and 37%, respectively.

Moreover, by increasing the temperature up to 40 °C, conversions around 85% were reached, while selectivity values remained around 50% with relatively good vanillin yields of 46%.

The observed increase of conversion by increasing the reaction temperature was expectable. However, in any case it is worth to remark that such values, and even the ones obtained for 25°C, are competitive with those ones reported either at higher temperatures, or prolonged reaction times and in the presence of a catalytic material.

**Example 3: Influence of $H_2O_2$ amount**

For this experiment, the influence of the amount of $H_2O_2$ was investigated at both 25°C and 40°C.

The conditions were similar to the ones described at Example 2, except the amount of $H_2O_2$. 20 mL of $H_2O_2$ was added into the mill after 5, 15, 30 and 60 min. Especially, the reactions have been performed under continuous-flow regimes and the temperature in the mill is of 25°C or 40°C. No catalyst is used for performing this example 3. After a residence time of 5, 15, 30 and 60 minutes in the mill, a final product is obtained and is recovered from the mill.

*Results*

At 25°C

*Table 7*

| Residence time (min) | Conversion rate (%) | Selectivity to vanillin (%) | Vanillin yield (%) |
|---|---|---|---|
| **5** | 80.6 | 56.5 | 45.5 |
| **15** | 88.0 | 49.8 | 43.8 |
| **30** | 92 | 50.9 | 46.8 |
| **60** | 89.2 | 48.9 | 43.6 |

At 40°C

*Table 8*

| Residence time (min) | Conversion rate (%) | Selectivity to vanillin (%) | Vanillin yield (%) |
|---|---|---|---|
| **5** | 60 | 49 | 29 |
| **30** | 55 | 40 | 22 |
| **60** | 48 | 33 | 16 |

As could be observed in Table 7, adding an excess of $H_2O_2$ could boost the reaction conversion rate up to 90% (residence time of 60 minutes), which is highly competitive value with those reported in the literature. In addition, selectivity values remained stable around 50% towards vanillin, with yields around 45%.

However, by performing the reaction at 40 °C adding an excess of $H_2O_2$, the reaction displayed lower values of conversion, selectivity and yield, in comparison with the similar experiments performed at room temperature. Those results could be most likely understood due to the possible decomposition of $H_2O_2$ at higher concentration under mechanochemical conditions and at 40 °C (Table 8).

**Example 4: Influence of the addition of a catalytic system**

For this experiment, the influence of a catalytic system was investigated at 25°C and 40°C.

The conditions were similar to the ones described at Example 3, except the presence of a nanocatalyst that is $Fe_2O_3$ (**500 mg).** Hence, 20 mL of $H_2O_2$ was added into the mill after 5, 15, 30 and 60 min sampling **(same as example 3)** Especially, the reactions have been performed under continuous-flow regimes and the temperature in the mill is of 25°C or 40°C. After a residence time of 5, 30 and 60 minutes in the mill, a final product is obtained and is recovered from the mill.

*Results*

At 25°C

*Table 9*

| Residence time (min) | Conversion rate (%) | Selectivity to vanillin (%) | Vanillin yield (%) |
|---|---|---|---|
| **5** | 52 | 75 | 39 |
| **30** | 60 | 73 | 43 |
| **60** | 73 | 74 | 54 |

At 40°C

*Table 10*

| Residence time (min) | Conversion rate (%) | Selectivity to vanillin (%) | Vanillin yield (%) |
|---|---|---|---|
| **5** | 49 | 77 | 38 |
| **30** | 55 | 76 | 42 |
| **60** | 77 | 74 | 57 |

Interestingly, these experiments exhibited lower conversion values, most likely attributed to the less efficient impacts of the microbeads, as a consequence of the presence of the solid catalyst in the reaction chamber (Tables 9 and 10). In turn, excellent selectivity values up to 75% were reached, which could be typically associated with the acidic nature of hematite phase of the employed $Fe_2O_3$ nanocatalyst. In any case, catalytic experiments in this case offer also an alternative for the synthesis of vanillin with higher selectivity, further simplifying purification processes. Even if this is an interesting possibility, it is worth to remark that similar vanillin yields could be reached also avoiding the use of catalyst and all the materials and cost involved in its prior preparation.

**Example 5: Comparative results on isoeugenol oxidation towards vanillin production reported in the literature**

Tables 11 shows the used parameters of different processes according to the prior art and Table 12 below shows the conversion rate, the selectivity to vanillin and the vanillin yield of these different processes according to the prior art ("MW: Microwave", CH: Conventional Heating, T: Temperature, t: time)

*Table 11*

| Comp.Ex | Prio art Ref. | Catalyst | Type of process | T (°C) | Time reaction |
|---|---|---|---|---|---|
| **A** | 1 | $PhI(OAc)_2$-NaY | MW | 130 | 5min |

(continued)

| Comp.Ex | Prio art Ref. | Catalyst | Type of process | T (°C) | Time reaction |
|---|---|---|---|---|---|
| **B** | 2 | Nocardia iowensiswholecells | CH | | |
| **C** | 3 | $TiO_2$ (P25 Degussa) | CH | | |
| **D** | 4 | CoTPyP/TN | CH | 50 | 24h |
| **E** | 5 | $(n\text{-}Bu)_4NVO_3$+PCA | CH | | |
| **F** | 6 | FeNMag-180 | CH | 25 | 24h |
| **G** | 7 | HMFeMo0.1 | CH | 80 | 5h |
| **H** | 8 | c@$Fe_2O_3$-700 °C | CH | 90 | 24h |
| **I** | 8 | c@$Fe_2O_3$-700 °C | MW | 90 | 30min |
| **J** | 9 | Co-hoba | CH | 25 | 24h |
| **K** | 9 | Ni-hoba | CH | 25 | 24h |
| **L** | 9 | Cu-hoba | CH | 25 | 24h |
| **M** | 10 | Ce-MCM-22(200) | CH | 60 | 1.5h |

The references correspond to the following:

1. H. Marquez Alvarez,D.P.Barbosa,A. TinocoFricks,D. A. G. Aranda, R. H.Vald∅s, O. A. C. Antunes, Org. Process Res. Dev. 2006, 10, 941-943.

2. R. Seshadri,A. S. Lamm,A. Khare, J. P. N. Rosazza, Enzyme Microb. Technol.2008, 43,486-494.

3. V. Augugliaro,G. Camera-Roda, V. Loddo, G. Palmisano, L. Palmisano, F. Parrino, M. A. Puma,Appl.Catal.B2012,111 -112,555-561.

4. Badria Adilina, T. Hara, N. Ichikuni, S. Shimazu ,J. Mol. Catal. A 2012,361-362,72-79.

5. E. V. Gusevskaya, L. Menini, L. A. Parreira, R. A. Mesquita, Y. N. Kozlov, G. B. Shul'pin, J. Mol. Catal. A 2012,363-364,140-147.

6. Marquez-Medina, M. D., Prinsen, P., Li, H., Shih, K., Romero, A. A., & Luque, R. ChemSusChem, 2018, 11(2), 389-396.

7. Neethu, P. P., Sreenavya, A., & Sakthivel, A. (2021). Molybdate Stabilized Magnesium-Iron Hydrotalcite Materials: Potential Catalysts for Isoeugenol to Vanillin and Olefin Epoxidation. Applied Catalysis A: General, 623, 118292.

8. Rodriguez-Padron, D., Munoz-Batista, M. J., Li, H., Shih, K., Balu, A. M., Pineda, A., Luque, R. ACS Sustain. Chem. Eng., 2019, 7(20), 17030-17038.

9. Srinivasapriyan, V. (2020). The surfactant-free bottom-up synthesis of ultrathin MOF nanosheets for the oxidation of isoeugenol to vanillin. Materials Advances, 1(3), 326-328.

10. Sahu, P., Ganesh, V., & Sakthivel, A. (2020). Oxidation of a lignin-derived-model compound: Iso-eugenol to vanillin over cerium containing MCM-22. Catalysis Communications, 145, 106099.

*Table 12*

| Comp.Ex | Conversion (%) | Selectivity to vanillin (%) | Vanillin Yield (%) |
|---|---|---|---|
| **A** | - | - | 43 |
| **B** | - | - | 60 |
| **C** | 99 | 9 | 8 |
| **D** | 99 | 72 | 71 |
| **E** | 71 | 70 | 50 |
| **F** | 85 | 57 | 49 |
| **G** | 86 | 83 | |

(continued)

| Comp.Ex | Conversion (%) | Selectivity to vanillin (%) | Vanillin Yield (%) |
|---|---|---|---|
| **H** | 51 | 14 | 7.1 |
| **I** | 60 | 25 | 15 |
| **J** | 18 | 90 | - |
| **K** | 23 | 92 | - |
| **L** | 10 | 82 | - |
| **M** | 63 | 75 | - |

As shown in this Table 12, the results obtained with the method according to the current invention are remarkably highly competitive with those reported in the literature so far and additionally, could be easily scale up.

Hence, as shown by the experiments above, the method according to the invention presents the following advantages:

- the use of a renewable feedstock derived from biomass, namely isoeugenol, for the synthesis of vanillin;
- the use of mild conditions to carry out the reactions (low temperature may be required);
- the use of continuous flow regimes and up-scale possibilities; and
- possibility of performing the reaction in absence of catalysts, therefore reducing of the use of required materials.

## Claims

**1.** Method for manufacturing vanillin that comprises at least the following steps:

(A) placing at least a starting material having the following formula (I):

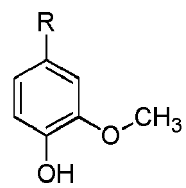

(I)

wherein R is selected from the group consisting in:

- a unsaturated straight or branched hydrocarbon group containing 1 to 3 carbon atoms, such as - CH=CH-$CH_3$;
- $R^1COOH$ wherein $R^1$ is an unsaturated, straight or branched hydrocarbon group containing 1 to 3 carbon atoms, such as -CH=CH-COOH,
- $R^2OH$ wherein $R^2$ is a unsaturated, straight or branched hydrocarbon group containing 1 to 3 carbon atoms, such as -$CH_2$-OH,

in a solvent with a liquid oxidant, referred to as "the starting mixture", the molar ratio of (liquid oxidant):( starting material) being greater than or equal to 2, preferably being within the range from 5 to 25, and in particular being withing the range from 5 to 15;

(B) milling said starting mixture at a temperature lower than or equal to 80°C, preferably lower than or equal to 50°C, in a three-dimensional liquid-phase microbead mill for a holding time lower than or equal to 180 min, preferably lower than or equal to 30 minutes, and in particular lower or equal to 10 minutes,

(C) recovering, at the outlet of the mill, a final product comprising at least vanillin and optionally other side-products.

**2.** Method according to claim 1, wherein the starting material is a natural compound or is derived from a natural compound that is preferably renewable.

**3.** Method according to claim 2, wherein the starting material is isoeugenol, vanillyl alcohol or ferulic acid, and is typically isoeugenol.

**4.** Method according to one of the preceding claims, wherein the starting solution at step (A) is free from any catalyst.

**5.** Method according any one of the preceding claims 1 to 4, wherein the liquid oxidant is hydrogen peroxide, urea hydrogen peroxide and tert-butyl hydroperoxide and is preferably hydrogen peroxide.

**6.** Method according to any one of the preceding claims, wherein the milling is carried out at an ambient temperature ranging from 15°C to 55°C, in particular ranging from 18°C to 50°C and in general is of the order of 20°C to 40°C.

**7.** Method according to one of the preceding claims, wherein the milling is carried out at a molar ratio of (liquid oxidant):(starting material) ranges from 5 to 15, in absence or presence of a catalytic system.

**8.** Method according to one of the preceding claims, wherein the microbeads are of spherical shape and have a mean diameter ranging from 0.05 mm to 4 mm, preferably from 0.2 mm to 3 mm, in particular from 0.3 mm to 2 mm and typically of the order of 0.5 mm to 1 mm.

**9.** Method according to one of the preceding claims, wherein the microbeads have a Vickers hardness measured as per the EN ISO 6507-1 standard greater than or equal to 900 HV1, preferably ranging from 900 HV1 to 1600 HV1, typically ranging from 1000 to 1400 HV1.

**10.** Method according to one of the preceding claims, wherein the three-dimensional microbead mill comprises at least:

- a stationary milling chamber of general cylindrical shape extending along a longitudinal axis XX, said chamber being filled at least, in part, with said microbeads and comprises: at a first end at least one inlet serving to introduce said starting mixture, and at a second end, an outlet including a separating means suitable for only discharging the vanillin and the optional other side-products formed in said chamber; and
- an agitator, arranged in the stationary milling chamber, presented in the form of an elongated rod along the longitudinal axis XX, said agitator being suitable for setting in motion the microbead/starting mixture assembly.

**11.** Method according to claim 10, wherein the microbeads represent, in volume, with respect to the total volume of the stationary chamber from 20% to 90%, preferably from 40% to 85%, in particular from 50% to 70%.

**12.** Method according to one of the preceding claims, wherein the mill operates in continuous mode.

**13.** Method according to one of claims 10 to 12, wherein the rotational speed of the agitator is greater than or equal to 100 revolutions per minute.

**14.** Method according to one of the preceding claims, wherein the microbeads are chosen among: ceramic microbeads, steel microbeads, tungsten carbide microbeads, glass microbeads or any of the combinations thereof.

**15.** Use of a three-dimensional microbead mill according to any one of the preceding claims 10 to 14 to synthetize vanillin.

Figure 1

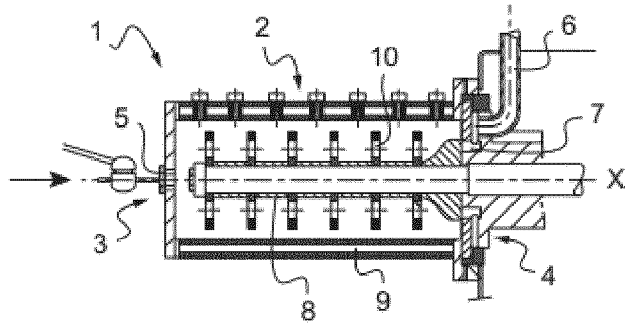


Figure 2

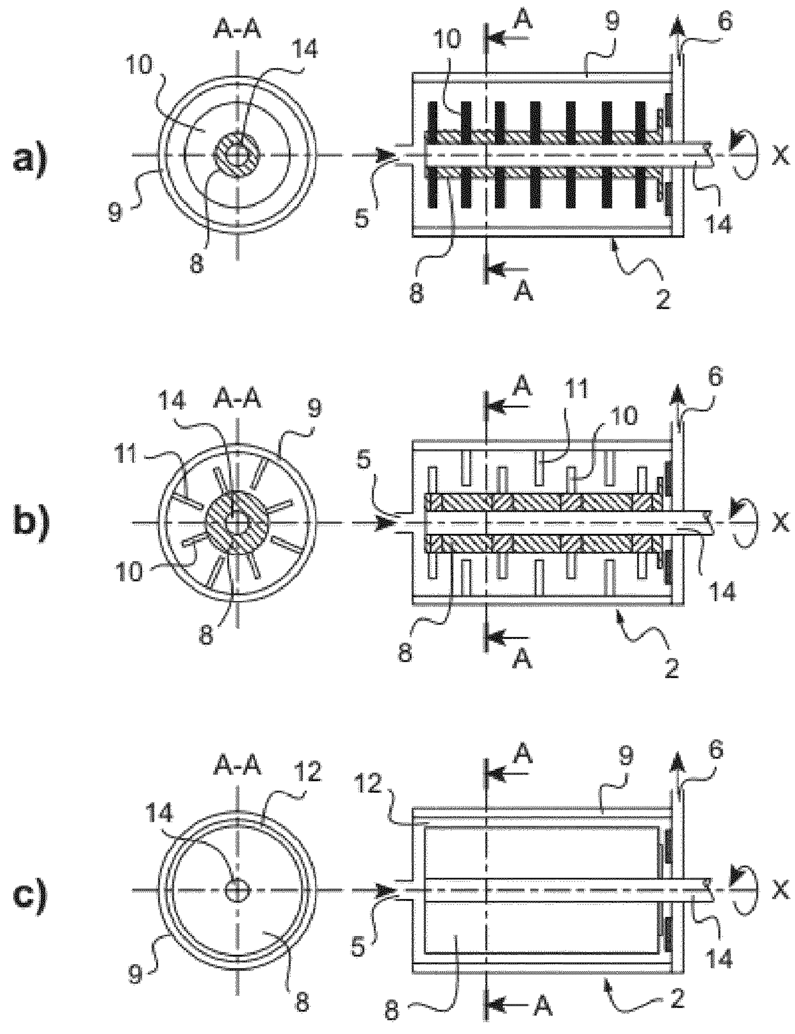

Figure 3

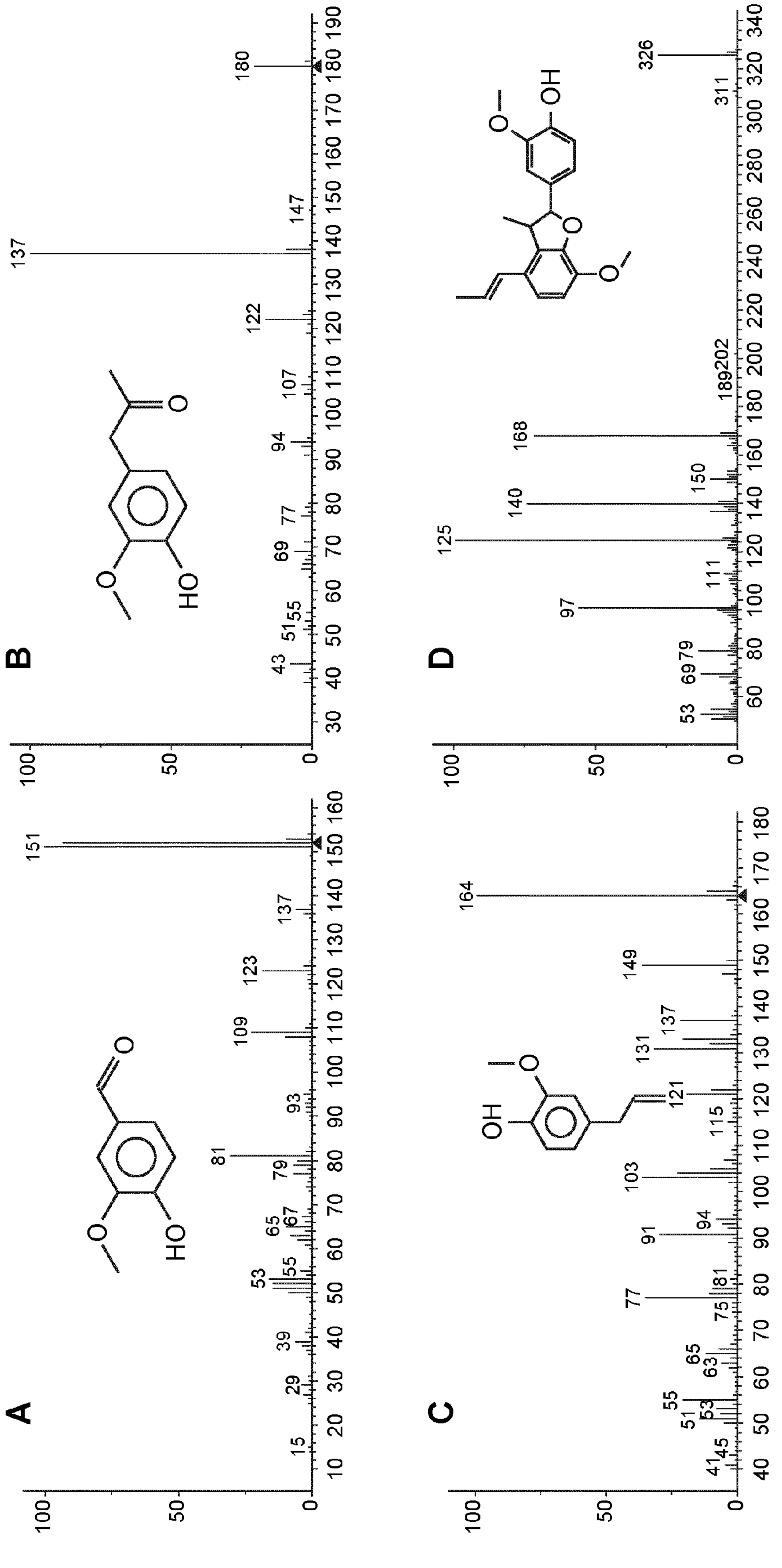
A
15 29 39 53 55 65 67 79 81 93 109 123 137 151
B
43 51 55 69 77 94 107 122 137 147 180
C
41 45 51 53 55 63 65 75 77 81 91 94 103 115 121 131 137 149 164
D
53 69 79 97 111 125 140 150 168 189 202 311 326

Europäisches Patentamt
European Patent Office
Office européen des brevets

# EUROPEAN SEARCH REPORT

Application Number
EP 22 30 6705

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOSSEINZADEH HOSSEINZADEH RAHMAN RAHMAN ET AL: "Pyridinium Chlorochromate Supported on Montmorillonite-KSF as a Versatile Oxidant under Ball Milling Conditions", ORGANIC PREPARATIONS AND PROCEDURES INTERNATIONAL: THE NEW JOURNAL FOR ORGANIC SYNTHESIS, ORGANIC PREPARATION AND PROCEDURES CO., NEWTON HIGHLANDS, MA, US, vol. 53, no. 5, 23 September 2021 (2021-09-23), pages 461-471, XP009544154, ISSN: 0030-4948, DOI: 10.1080/00304948.2021.1944733<br>* table 2 *<br>* the whole document *<br>----- | 1-15 | INV.<br>C07C45/28<br>C07C47/58 |
| | | | TECHNICAL FIELDS SEARCHED (IPC) |
| | | | C07C |

The present search report has been drawn up for all claims

| Place of search | Date of completion of the search | Examiner |
|---|---|---|
| The Hague | 3 May 2023 | Tabanella, Stefania |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019228983 A
- FR 1854592
- US 5597126 A


### Non-patent literature cited in the description


- *CHEMICAL ABSTRACTS,* 121-33-5
- **RODRIGUEZ-PADRON, D. ; MUNOZ-BATISTA, M. J. ; LI, H. ; SHIH, K. ; BALU, A. M. ; PINEDA, A. ; LUQUE, R.** *ACS Sustain. Chem. Eng.,* 2019, vol. 7 (20), 17030-17038
- **RODRIGUEZ-PADRON et al.** *ACS Sustain.Chem.Eng.,* 2019, vol. 7 (20), 17030-17038
- *CHEMICAL ABSTRACTS,* 124-43-6
- **H. MARQUEZ ALVAREZ ; D.P.BARBOSA ; A. TINOCOFRICKS ; D. A. G. ARANDA ; R. H.VALD∅S ; O. A. C. ANTUNES.** *Org. Process Res. Dev.,* 2006, vol. 10, 941-943
- **R. SESHADRI ; A. S. LAMM ; A. KHARE ; J. P. N. ROSAZZA.** *Enzyme Microb. Technol.,* 2008, vol. 43, 486-494
- **V. AUGUGLIARO ; G. CAMERA-RODA ; V. LODDO ; G. PALMISANO ; L. PALMISANO ; F. PARRINO ; M. A. PUMA.** *Appl.Catal.B,* 2012, vol. 111 -112, 555-561
- **BADRIA ADILINA ; T. HARA ; N. ICHIKUNI ; S. SHIMAZU.** *J. Mol. Catal. A,* 2012, vol. 361 (362), 72-79
- **E. V. GUSEVSKAYA ; L. MENINI ; L. A. PARREIRA ; R. A. MESQUITA ; Y. N. KOZLOV ; G. B. SHUL'PIN.** *J. Mol. Catal. A,* 2012, vol. 363 (364), 140-147
- **MARQUEZ-MEDINA, M. D. ; PRINSEN, P. ; LI, H. ; SHIH, K. ; ROMERO, A. A. ; LUQUE, R.** *ChemSusChem,* 2018, vol. 11 (2), 389-396
- **NEETHU, P. P. ; SREENAVYA, A. ; SAKTHIVEL, A.** Molybdate Stabilized Magnesium-Iron Hydrotalcite Materials: Potential Catalysts for Isoeugenol to Vanillin and Olefin Epoxidation. *Applied Catalysis A: General,* 2021, vol. 623, 118292
- **SRINIVASAPRIYAN, V.** The surfactant-free bottom-up synthesis of ultrathin MOF nanosheets for the oxidation of isoeugenol to vanillin. *Materials Advances,* 2020, vol. 1 (3), 326-328
- **SAHU, P. ; GANESH, V. ; SAKTHIVEL, A.** Oxidation of a lignin-derived-model compound: Iso-eugenol to vanillin over cerium containing MCM-22. *Catalysis Communications,* 2020, vol. 145, 106099